# EUROPÄISCHE PATENTSCHRIFT

(11) **EP 1 260 186 B1**
(45) Veröffentlichungstag und Bekanntmachung des Hinweises auf die Patenterteilung: **28.02.2007**
(21) Anmeldenummer: 02011116.7
(22) Anmeldetag: 18.05.2002
(51) Int. Cl.: A61B 17/34, A61N 1/05

(54) **Kombinationsnadel für periphere Nervenblockaden**
Needle assembly for blocking peripheral nerves
Ensemble aiguille pour le blocage des nerfs périphériques

(30) Priorität: 22.05.2001 DE 20108558 U
(43) Veröffentlichungstag der Anmeldung: 27.11.2002
(73) Patentinhaber: Transmed Medizintechnik GmbH & Co. KG, 33181 Bad Wünnenberg (DE)
(72) Erfinder: Krebs, Peter K, Dr., 78048 Villingen-Schwenningen (DE)
(74) Vertreter: Neymeyer, Franz

(56) Entgegenhaltungen:
- EP-A- 0 823 239
- DE-A- 3 508 013
- DE-A- 3 602 219
- DE-A- 19 640 670
- US-A- 3 682 162
- US-A- 5 885 219

## Beschreibung

Die Erfindung betrifft eine Kombinationsnadel für periphere Nervenblockaden, bestehend aus einer Kunststoffkanüle und einem im wesentlichen zylindrischen, massiven Stahlmandrin, der mit einer schneidkantenfreien Stechspitze und mit einem Griffteil versehen ist, das einen elektrischen Steckkontakt für den Anschluß eines Elektrostimulators aufweist, wobei der Stahlmandrin derart in die Kanüle einführbar ist, daß seine Stechspitze aus deren vorderem Ende herausragt oder zumindest mit diesem bündig ist.

Eine bipolare Elektrode zum Auffinden nervaler Strukturen ist aus DE 196 40 670 A1 bekannt. Diese besteht aus einer Kanüle mit einer seitliche Öffnung zum Injizieren von Pharmaka und mit zwei außenseitig mit einem axialen Abstand voneinander angebrachten Elektroden. Dabei besteht eine Elektrode aus einem in einer äußeren axialen Nut isoliert verlaufenden elektrischen Leiter und die zweite Elektrode aus der freien Metallspitze der Kanüle.

Bei einer anderen sog. Neuroelektrode, die aus DE 36 02 219 bekannt ist und die zur Rückenmarkstimulation benutzt werden kann, ist ein Spülkanal zum Instillieren von dünnflüssigen Lösungen vorgesehen, der radiale Öffnungen aufweist, die in der Nähe eines frei liegenden Metalltips eines Pols mit einem axialen Abstand zueinander angeordnet sind. Hierbei sind auch zwei in axialem Abstand voneinander angeordnete Pole (Elektroden) vorgesehen. Es ist aber nicht ersichtlich, wie diese ausgebildet und gegeneinander isoliert sind bzw. wie sie elektisch getrennt mit den dafür am Handgriff der Kanüle angeordneten elektrischen Steckanschlüssen verbunden sind.

Die aus DE-35 08 013 C2 bekannte Kobinationsnadel, zu der ein massiver Stahlmandrin gehört, gemäß dem Oberbegriff des ersten Anspruch 5, wird für die Anästhesie und Analgesie des Armes verwendet, wobei bevorzugt eine Blockade des Plexus-Brachialis im axillären Bereich durchgeführt wird.

Diese bekannte Kombinationsnadel zeichnet sich durch besondere Gestaltungen der Stechspitze des aus einem massiven Stab bestehenden Stahlmandrins aus, die geeignet sind, die aus festem Gewebe bestehende Nervenhülle, auch Nervenscheide oder Fascie genannt, mit möglichst geringem Kraftaufwand zu durchstoßen und bei denen die Gefahr von Nervenverletzungen auf ein Minimum reduziert ist.

Außerdem soll die jeweilige Lage der Nadelspitze exakt lokalisierbar sein. Während die erstgenannten Forderungen allein durch die Form der Stechspitze des Stahlmandrins erfüllt werden können, erfolgt die Lokalisierung der Stechspitze am Nervenstrang durch eine Elektrostimulation, die durch Anlegen einer elektrischen Spannung bestimmter Stärke und kurzer Dauer durchgeführt wird. Dazu ist bei der bekannten Kombinationsnadel an dem mit dem Handgriff versehenen Ende des Stahlmandrins eine Steckerbuchse vorgesehen. Anhand einer auf der Mantelfläche der Kunststoffkanüle vorgesehenen Längenskala kann ein Anästhesist zwar ablesen, wie tief die Kanüle in die Haut eingedrungen ist. Er kann aber daran nicht feststellen, wie weit die Kanülenspitze in der Gefäß-Nerven-Scheide liegt, weil der Abstand zwischen der Gefäß-Nerven-Scheide und der Hautoberfläche bzw. der Einstichstelle von Patient zu Patient verschieden ist.

Diese Steckerbuchse ist aber nur einpolig ausgebildet, d.h. es kann nur ein Pol einer Spannungsquelle an den Stahlmandrin angeschlossen werden. Der zweite notwendige Pol ist an eine Elektrode angeschlossen, die bei der Verwendung dieser bekannten Kombinationsnadel irgendwo außenseitig am Körper des betreffenden Patienten angelegt wird, so daß in jedem Falle zwischen der Spitze des Stahlmandrins, welche die eine Elektrode bildet und der zweiten Elektrode, die außenseitig am Körper des Patienten an einer günstigen Stelle befestigt wird, immer ein relativ großer Abstand besteht.

Dadurch ergibt sich der Nachteil, daß in jedem Falle größere Körperpartien von der elektrischen Wirkung der Elektrostimulation erfaßt werden. Dies kann bei Patienten, die mit implantierten elektronischen Geräten, beispielsweise einem Herzschrittmacher, versehen sind, zu schädlichen Ergebnissen führen, die unbedingt vermieden werden sollten.

Der Erfindung liegt die Aufgabe zugrunde, eine Kombinationsnadel der eingangs genannten Art zu schaffen, bei der die beiden zu einer Elektrostimulation erforderlichen Elektroden an der Stechspitze des Stahlmandrins sehr eng beieinander liegen und die elektrischen Wirkungen der Stimulation auf den Körperbereich beschränkt ist, wo die Stimulation, stattfinden soll.

Gelöst wird diese Aufgabe erfindungsgemäß dadurch, daß der Stahlmandrin zweipolig ausgebildet ist, wobei ein Pol von der Stechspitze (42) des Stahlmandrins (10) gebildet wird und der zweite Pol aus einer leitenden Mantelschicht (13) besteht, die auf einer die Mantelfläche des Stahlmandrins (10) geschlossen umschließenden Isolierstoffschicht (14) aufgebracht ist, und wobei die beiden Pole mit einer zweipoligen elektrischen Steckkontakteinrichtung (21, 37) verbunden sind.

Mit der erfindungsgemäß ausgestalteten Kombinationsnadel ist erstmals die Möglichkeit geschaffen, die Wirkung einer Elektrostimulation auf den Bereich der Stechspitze des Stahlmandrins zu konzentrieren und auch Körperpartien der näheren Umgebung davon zu verschonen. Es wird dadurch eine zielgenauere Nervenstimulation ermöglicht. Außerdem wird zugleich auch eine Möglichkeit eröffnet, eine solche Kombinationsnadel auf einfache Weise herzustellen und zu handhaben.

Dabei ist es mit der Ausgestaltung nach Anspruch 2 auch möglich, sehr dünne Stahlmandrine bzw. dünne Kombinationsnadeln herzustellen, weil die Isolierstoffschicht und die leitende Mantelschicht sehr dünn sein können. Die Dicke einer solchen Schicht kann beispielsweise im Bereich von 0,005 mm bis 0,2 mm liegen.

Weitere vorteilhafte Ausgestaltungen der Erfindung sind Gegenstand der Ansprüche 3 bis 7, wobei diese Ausgestaltungen insbesondere den Vorteil haben, daß sie die Möglichkeit eröffnen, wenigstens zum Teil handelsübliche Steckverbindungen zu verwenden und die Kontakt gebenden Verbindungen zwischen den leitenden Teilen des Stahlmandrins und des Steckerteils auf rein mechanische Weise, also ohne Löten oder Schweißen, zuverlässig und auch mit der notwendigen Festigkeit herstellen zu können.

Anhand der Zeichnung wird im folgenden ein Ausführungsbeispiel der Erfindung näher erläutert. Es zeigt:
- Fig. 1: die Kombinationsnadel mit aufgesetzter Kunststoffkanüle im Schnitt;
- Fig. 2: in isometrischer Explosionsdarstellung die Kombinationsnadel, bestehend aus Stahlmandrin mit Handgriff und Kunststoffkanüle mit Handgriff sowie mit einer elektrischen Steckerbuchse;
- Fig. 3: den Griffteil des Stahlmandrins in stark vergrößerter Schnittdarstellung;
- Fig. 4: einen noch stärker vergrößerten Schnitt IV-IV aus Fig. 3;
- Fig. 5: im gleichen Maßstab einen Schnitt V-V aus Fig. 3;
- Fig. 6: ebenfalls im gleichen Maßstab einen Schnitt VI-VI aus Fig. 3;
- Fig. 7a-7f: verschieden geformte Stechspitzen des Stahlmandrins in Seitenansicht.

Die in der Zeichnung dargestellte Kombinationsnadel für periphere Nervenblockaden besteht aus einer Kunststoffkanüle 1 und einem zylindrischen, massiven Stahlmandrin 10. Die Kunststoffkanüle 1 hat einen Durchmesser **D2** von etwa 0,8 mm bis 1,3 mm, während der Durchmesser **D1** des Stahlmandrins 10 abgestimmt auf den Innendurchmesser der Kunststoffkanüle 1 etwa 0,6 mm bis 1 mm betragen kann.

Die Kunststoffkanüle 1 ist mit einem Handgriff 2 versehen, der ebenfalls aus Kunststoff besteht und der einen rückseitig offenen Hohlraum 3 aufweist. Dieser Hohlraum 3 ist konisch gestaltet, so daß er einen sog. Luer-Konus 4 passend aufnehmen kann, der Bestandteil eines Griffteils 11 des Stahlmandrins 10 ist. Die Kunststoffkanüle 1 und ihr Handgriff 2 sind in üblicher Weise fest miteinander verbunden.

Der Stahlmandrin 10 besteht aus einem massiven Stahlkern 12, der elektrisch leitend den einen Pol bzw. die eine Elektrode bei der Elektrostimulation bildet. Der Stahlmandrin 10 ist dadurch zweipolig ausgestaltet, daß der zweite Pol bzw. die zweite Elektrode aus einer leitenden metallischen Mantelschicht 13 gebildet ist, die auf einer die Mantelfläche des Stahlkerns 12 geschlossen umschließenden Isolierstoffschicht 14 aufgebracht ist. Dabei besteht die Isolierstoffschicht 14 aus einem Isolierlack und die leitende Mantelschicht 13 aus einem galvanisch aufgetragenen Metallbelag, vorzugsweise Nickel, Chrom oder Gold.

Das hintere Ende 15 des Stahlkerns, das von der leitenden Mantelschicht 13 und der Isolierstoffschicht 14 befreit ist, ist in einer zentralen Axialbohrung 9 eines im wesentlichen zylindrischen Metallteils 16 kontaktierend befestigt. Dieses Metallteil 16 ist einstückiger Bestandteil eines Steckerstiftes 17. Der Steckerstift 17 ist koaxial zu einer Mittelachse 18 in einer Isolierstoffbuchse 19 einer metallenen Steckerhülse 20 festsitzend angeordnet, und er besitzt eine Kontaktspitze 21, die frei in einen zylindrischen, stirnseitig offenen Hohlraum 22 der Isolierstoffbuchse 19 ragt.

Die zur Mittelachse 18 ebenfalls koaxiale Isolierstoffbuchse 19 ist festsitzend in der Steckerhülse 20 angeordnet. Die Steckerhülse 20 ist mit einem Gewindeschaft 23 versehen, der in ein passendes Innengewinde 24 einer metallenen Kupplungshülse 25 soweit eingeschraubt ist, daß ein vorzugsweise als Sechskant ausgebildeter Flanschring 26 auf der stirnseitigen Ringfläche 27 der Kupplungshülse 25 aufsitzt.

Die Kupplungshülse 25 und die Steckerhülse 20 lassen sich somit leicht zu dem Griffteil 11 zusammenfügen. Die Kupplungshülse 25 ist vorderseitig mit dem bereits erwähnten Luer-Konus 4 versehen, an den sich einstückig ein im Durchmesser verjüngter Hülsenabschnitt 28 anschließt. Vorderseitig ist die Kupplungshülse 25 mit einer auf den Durchmesser **D1** des Stahlmandrins 10 abgestimmten zentralen Axialbohrung 29 versehen, in welcher der hintere Teil 30 des Stahlmandrins 10 mit der leitenden Mantelschicht 13 und der Isolierstoffschicht 14 aufgenommen ist. Zur kontaktierenden Befestigung des Stahlmandrins 10 in dieser Axialbohrung 29 ist der verjüngte Hülsenabschnitt 28 unter Bildung von Sicken 31 und 32 radial mit dem Stahlmandrin 10 verpreßt, so daß nicht nur eine kontaktierende Verbindung zwischen der leitenden Mantelschicht 13 des Stahlmandrins 10 und der Kupplungshülse 25 sondern auch eine zugfeste Verbindung zwischen diesen Teilen entsteht. Die Gewindeverbindung 23/24 ist dabei unbedingt erforderlich.

Auf die gleiche Weise, d.h. durch radiales Verpressen des Metallteils 16, ist auch das hintere Ende 15 des Stahlkerns 12 mit dem Steckerstift 17 kontaktierend und zugfest verbunden.

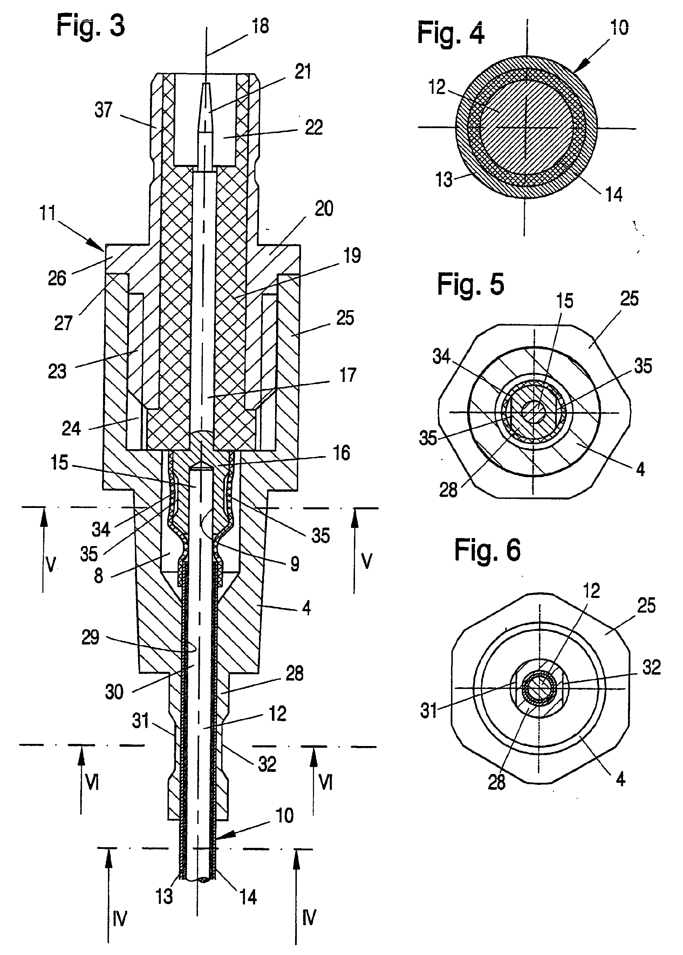

Die durch das axiale Verpressen am Metallteil 16 entstandenen Sicken 35 und 36 sind auch in der Fig. 5 sichtbar.

Auf das Metallteil 16 ist ein elastisches Schlauchstück aus Isoliermaterial aufgezogen, das auch das hintere Ende der leitenden Mantelschicht 13 und der Isolierstoffschicht 14 umschließt.

Zur freien Aufnahme des mit dem elastischen Schlauchstück 34 überzogenen Metallteils 16 ist die Kupplungshülse 25 mit einer zylindrischen, am vorderen Ende konisch auslaufenden Bohrung 8 versehen.

Am hinteren Ende besitzt die Steckerhülse 20 einen Rohransatz 37, der den hinteren Abschnitt der Isolierstoffbuchse umschließt und zur rastenden Aufnahme einer koaxialen, mit zwei Steckkontakten und zwei Anschlußleitungen 38, 39 versehenen, handelsüblichen Steckerbuchse 40 dient.

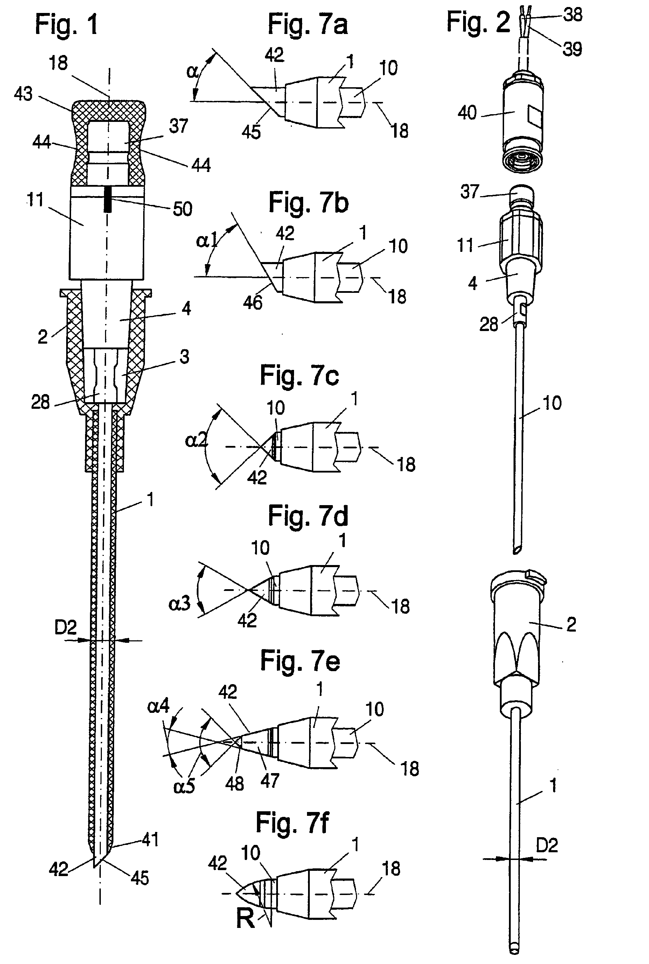

Die Kunststoffkanüle 1 ist in ihrer Länge so bemessen, daß sie, wie in Fig. 1 dargestellt ist, mit ihrem vorderen konisch verjüngten Ende 41 bis knapp an die Stechspitze 42 des Stahlmandrins 10 reicht, wenn ihr Handgriff 2 festsitzend auf den Luer-Konus 4 geschoben ist.

Für die Gestaltung der Stechspitze sind die bekannten, bereits in der DE 35 08 013 C2 beschriebenen Grundsätze zu beachten. Solche Stechspitzen, die den gestellten Anforderungen entsprechen, sind in den Fig. 7a bis 7f beispielsweise dargestellt.
Die Stechspitze 42 der Fig. 7a ist einseitig so angeschliffen, daß eine Anschlifffläche 45 entsteht, die zur Mittelachse 18 einen Keilwinkel α von 45° bildet. Diese Anschlifffläche 45 hat die geometrische Form einer Ellipse, die ausschließlich von der zylindrischen Mantelfläche des Stahlmandrins 10 bzw. von der leitenden Mantelschicht 13 gebildet ist und keinerlei Facetten aufweist. Scharfe Schneidkanten sind bei dieser Stechspitze 42 zu vermeiden.

In Fig. 7b beträgt der Keilwinkel α1 der einseitigen Anschliffläche 46 60°. Diese Stechspitze 42 ist somit stumpfer als die Stechspitze 42 der Fig. 7a. Sie weist ebenfalls keine Facetten und scharfen Schneidkanten auf.

Bei diesen einseitig angeschliffenen Stechspitzen 42 des Stahlmandrins 10 ist es für den Anwender nach dem Einstechen der Kombinationsnadel wichtig zu wissen, welche Lage die Anschlifffläche 45, 46 jeweils einnimmt. Es ist deshalb am Griffteil eine Markierung 50 (Fig. 1) vorgesehen, die z.B. aus einer Kerbe bestehen kann und an welcher der Anwender die Drehlage der Anschlifffläche 45, 46 erkennen kann.

Die in Fig. 7c dargestellte Stechspitze 42 besteht aus einem geraden spitzen Kegel, dessen Kegelwinkel α2 90° beträgt.

Bei der ebenfalls kegelförmigen Stechspitze 42 der Fig. 7d beträgt der Kegelwinkel α3 nur 60°; die Kegelspitze ist jedoch kugelabschnittförmig abgerundet, wobei der Rundungsradius etwa dem halben Durchmesser des Stahlmandrins 10 entspricht.

Fig. 7e zeigt eine Stechspitze 42, die zweistufigkegelförmig ausgebildet ist und einen schlankeren Abschnitt 47 mit einem Kegelwinkel α4 von 30° und einem stumpferen Endteil 48 mit einem Kegelwinkel α5 von 120° aufweist.

Schließlich zeigt Fig. 7f eine Stechspitze 42, die als bauschiger Kegel ausgebildet ist, dessen Krümmungsradius **R** etwa dem 1,5-fachen Durchmesser **D1** des Stahlmandrins 10 entspricht.

Es versteht sich von selbst, daß die zweipolige Gestaltung des Stahlmandrins 10 und der Steckkontakteinrichtung am Griffteil 11 auf die Formgebung der Stechspitze 42 keinen einschränkenden Einfluß haben.

Die Zweipoligkeit der beschriebenen Kombinationsnadel ist somit dadurch gegeben, daß der Kernteil 12 des Stahlmandrins 10 elektrisch leitend nur mit dem Steckerstift 17 und die leitende Mantelschicht 13 des Stahlmandrins 10 über die Kupplungshülse 25 elektrisch leitend nur mit der Steckerhülse 20 und deren Rohransatz 37 elektrisch leitend verbunden sind. Die leitende Mantelschicht 13 ist gegen den Kernteil 12 des Stahlmandrins 10 durch die Isolierstoffschicht 14 isoliert; die Kupplungshülse 25 und die Steckerhülse 20, die beide aus leitendem Material bestehen und miteinander Kontakt haben, sind durch die Isolierstoffbuchse 19 gegen den Steckerstift 17 isoliert.

Die Handhabung der erfindungsgemäßen Kombinationsnadel wird durch die Zweipoligkeit in keiner Weise beeinträchtigt oder verändert, wenn man von eingangs erwähnten Vorteilen dieser Zweipoligkeit bei der Anwendung der Elektrostimulation absieht, deren fokusierende lokale Begrenzung auf den Bereich der Nadelspitze gegenüber der bekannten Kombinationsnadel einen erheblichen Fortschritt darstellt.

Um die Kombinationsnadel besser handhaben zu können und um zugleich die elektrischen Kontaktelemente des Griffteils 11, nämlich den Rohransatz 37 und die Kontaktspitze 21 gegen schädliche äußere Einflüsse zu schützen, ist eine Schutzkappe 43 aus Kunststoff vorgesehen. Diese Schutzkappe 43 leicht abnehmbar auf den Rohransatz 37 aufgesteckt, so daß er diesen vollständig umschließt. Damit er mit den Fingern beim Aufstecken und Abziehen gut gehalten werden kann, ist er an seinem Umfang mit Griffmulden 44 versehen.

## Patentansprüche

1. Kombinationsnadel für periphere Nervenblockaden, bestehend aus einer Kunststoffkanüle (1) und einem im wesentlichen zylindrischen, massiven Stahlmandrin (10), der mit einer schneidkantenfreien Stechspitze und mit einem Griffteil (11) versehen ist, das einen elektrischen Steckkontakt (21) für den Anschluß eines Elektrostimulators aufweist, wobei der Stahlmandrin (10) derart in die Kanüle (1) einführbar ist, daß seine Stechspitze (42) aus deren vorderem Ende herausragt oder zumindest mit diesem bündig ist, **dadurch gekennzeichnet, daß** der Stahlmandrin (10) zweipolig ausgebildet ist, wobei die Stechspitze (42) den einen Pol bildet und der zweite Pol aus einer leitenden Mantelschicht (13) besteht, die auf einer die Mantelfläche des Stahlmandrins (10) geschlossen umschließenden Isolierstoffschicht (14) aufgebracht ist und die beiden Pole mit einer zweipoligen elektrischen Steckkontakteinrichtung (21, 37) verbunden sind,

2. Kombinationsnadel nach Anspruch 1, **dadurch gekennzeichnet, daß** die Isolierstoffschicht (14) aus einem Isolierlack und die leitende Mantelschicht (13) aus einem galvanisch aufgetragenen Metallbelag besteht.

3. Kombinationsnadel nach Anspruch 2, **dadurch gekennzeichnet, daß** das hintere Ende (15) des Stahlkerns von der leitenden Mantelschicht (13) und der Isolierstoffschicht (14) befreit in einer zentralen Axialbohrung (16) eines in einer Isolierstoffbuchse (19) einer metallenen Steckerhülse (20) gelagerten Stekkerstiftes (17) kontaktierend befestigt ist und daß der hintere Abschnitt der leitenden Mantelschicht des Stahlmandrins kontaktierend in einer zentralen Axialbohrung (28) einer mit einem Luer-Steckkonus (4) versehenen Kupplungshülse (25) befestigt ist.

4. Kombinationsnadel nach Anspruch 3, **dadurch gekennzeichnet, daß** die Steckerhülse (20) und die Kupplungshülse (25) in koaxialer Anordnung miteinander verschraubt sind.

5. Kombinationsnadel nach Anspruch 3, **dadurch gekennzeichnet, daß** sich die das hintere Ende (15) des Stahlkerns (12) aufnehmende Axialbohrung (16) in einem einstückig mit dem Steckerstift (17) verbundenen und axial außerhalb der Isolierstoffbuchse (19) liegenden Fortsatz (16) befindet, der mit dem Stahlkern (12) radial verpreßt ist.

6. Kombinationsnadel nach Anspruch 3, **dadurch gekennzeichnet, daß** der Luer-Steckkonus (4) einen im Durchmesser verjüngten Hülsenabschnitt (28) aufweist, der, nur die leitende Mantelschicht (13) kontaktierend, mit dem Stahlmandrin (10) radial verpreßt ist.

7. Kombinationsnadel nach Anspruch 3, **dadurch gekennzeichnet, daß** die Steckerhülse (20) zur rastenden Aufnahme einer koaxialen, mit zwei Steckkontakten und zwei Anschlußleitungen (38, 39) versehenen Steckerbuchse (40) eine die Kontaktspitze (21) des Steckerstiftes (17) und die Isolierstoffbuchse (19) umschließenden Rohransatz (37) aufweist.

## Claims

1. Combination needle for peripheral nerve blocks, consisting of a plastics material cannula (1) and a substantially cylindrical solid steel mandrel (10) which is provided with a cutting edge-free pricking tip and a handle part (11), which has an electrical plug contact (21) for the connection of an electrostimulator, wherein the steel mandrel (10) can be introduced into the cannula (1) in such a way that its pricking tip (42) protrudes from the front end of the cannula or is at least flush therewith, **characterised in that** the steel mandrel (10) has a two-pole design, wherein the pricking tip (42) forms one pole and the second pole consists of a conductive jacket layer (13), which is applied on an insulating layer (14) which encloses the lateral surface of the steel mandrel (10) in a closed manner, and the two poles are connected to a two-pole electrical plug contacting means (21, 37).

2. Combination needle according to claim 1, **characterised in that** the insulating material layer (14) consists of an insulating varnish and the conductive jacket layer (13) consists of a metal coating applied by electroplating.

3. Combination needle according to claim 2, **characterised in that** the rear end (15) of the steel core, freed from the conductive jacket layer (13) and the insulating material layer (14), is fastened in a contacting manner in a central axial bore (16) of a plug pin (17) mounted in an insulating material socket (19) of a metallic plug socket (20) and **in that** the rear section of the conductive jacket layer of the steel mandrel is fastened in a contacting manner in a central axial bore (28) of a coupling sleeve (25) provided with a Luer's cone (4).

4. Combination needle according to claim 3, **characterised in that** the plug socket (20) and the coupling sleeve (25) are screwed together in a coaxial arrangement.

5. Combination needle according to claim 3, **characterised in that** the axial bore (16) receiving the rear end (15) of the steel core (12) is located in a projection (16) integrally connected to the plug pin (17) and located axially outside the insulating material socket (19), which projection is radially pressed together with the steel core (12).

6. Combination needle according to claim 3, **characterised in that** the Luer's cone (4) has a sleeve section (28) which is tapered with regard to diameter, which, contacting only the conductive jacket layer (13), is radially pressed together with the steel mandrel (10).

7. Combination needle according to claim 3, **characterised in that** the plug sleeve (20) has a tubular attachment (37) enclosing the contact tip (21) of the plug pin (17) and the insulating material socket (19) for latching receiving of a coaxial plug socket (40) provided with two plug contacts and two connection lines (38, 39).

## Revendications

1. Aiguille combinée pour les blocs nerveux périphériques, composée d'une canule en matière plastique (1) et d'un mandrin en acier massif (10), essentiellement cylindrique, qui est muni d'une pointe perforante sans arête coupante et d'un manche (11) qui présente un contact à fiche électrique (21) pour le raccordement d'un électrostimulateur, le mandrin en acier (10) pouvant être introduit dans la canule (1) de manière que sa pointe perforante (42) dépasse de son extrémité avant ou au moins vienne en affleurement avec celle-ci, **caractérisée en ce que** le mandrin en acier (10) est bipolaire, la pointe perforante (42) formant le premier pôle et le deuxième pôle étant formé par une couche d'enveloppe conductrice (13) qui est appliquée sur une couche isolante (14) entourant de manière fermée la surface d'enveloppe du mandrin en acier (10) et les deux pôles étant connectés par un dispositif de contact à fiche électrique bipolaire (21, 37).

2. Aiguille combinée selon la revendication 1, **caractérisée en ce que** la couche isolante (14) est composée d'un vernis isolant et la couche d'enveloppe conductrice (13) d'un revêtement métallique appliqué par galvanisation.

3. Aiguille combinée selon la revendication 2, **caractérisée en ce que** l'extrémité arrière (15) du noyau en acier est fixée, en dehors de la couche d'enveloppe conductrice (13) et de la couche isolante (14), en faisant contact dans un alésage axial central (16) d'une broche de connexion (17) logée dans un manchon isolant (19) d'une douille de connexion métallique (20) et que la partie arrière de la couche d'enveloppe conductrice du mandrin en acier est fixée en faisant contact dans un alésage axial central (28) d'une douille d'accouplement (25) muni d'un cône Luer (4).

4. Aiguille combinée selon la revendication 3, **caractérisée en ce que** la douille de connexion (20) et la douille d'accouplement (25) sont vissées ensemble dans une disposition coaxiale.

5. Aiguille combinée selon la revendication 3, **caractérisée en ce que** l'alésage axial (16) recevant l'extrémité arrière (15) du noyau en acier (12) se trouve dans un prolongement (16) assemblé en une seule pièce avec la broche de connexion (17), situé axialement en dehors du manchon isolant (19) et serti radialement avec le noyau en acier (12).

6. Aiguille combinée selon la revendication 3, **caractérisée en ce que** le cône Luer (4) présente une partie de douille (28) de diamètre réduit qui est sertie radialement avec le mandrin en acier (10) en faisant contact seulement avec la couche d'enveloppe conductrice (13).

7. Aiguille combinée selon la revendication 3, **caractérisée en ce que** la douille de connexion (20) présente, pour la réception par encliquetage d'un connecteur femelle (40) coaxial, muni de deux contacts à fiche et de deux câbles de connexion (38, 39), un embout tubulaire (37) entourant la pointe de contact (21) de la broche de connexion (17) et le manchon isolant (19).
